Europäisches Patentamt

European Patent Office

Office européen des brevets

(11) Veröffentlichungsnummer: **0 100 910**
**B1**

(12) **EUROPÄISCHE PATENTSCHRIFT**

(45) Veröffentlichungstag der Patentschrift:
02.11.88

(51) Int. Cl.⁴: **A 61 K 31/70 //**
**(A61K31/70, 31:685, 31:66)**

(21) Anmeldenummer: **83106935.6**

(22) Anmeldetag: **15.07.83**

(54) **Lungensurfactant.**

(30) Priorität: **05.08.82 DE 3229179**

(43) Veröffentlichungstag der Anmeldung:
**22.02.84 Patentblatt 84/8**

(45) Bekanntmachung des Hinweises auf die Patenterteilung:
**02.11.88 Patentblatt 88/44**

(84) Benannte Vertragsstaaten:
**AT BE CH DE FR GB IT LI LU NL SE**

(56) Entgegenhaltungen:
**EP-A-0 050 793**
**DE-A-2 900 300**
**GB-A-2 050 832**

**CHEMICAL ABSTRACTS, Band 92, Nr. 3, 21. Januar 1980, Seite 62, Nr. 15574p, Columbus, Ohio, US; M. IKEGAMI u.a.: "Restoration of lung pressure-volume characteristics with various phospholipids" & PEDIATR. RES. 1979, 13 (6), 777-80**

(73) Patentinhaber: **A. Nattermann & Cie. GmbH, Nattermannallee 1, D-5000 Köln 30 (DE)**

(72) Erfinder: **Röntgen- Odenthal, Renate, Wasserackerstrasse 16, D-7800 Freiburg-Littenweiler (DE)**
Erfinder: **Dürr, Manfred, Dr. Dipl.- Chem., Am blauen Stein 10, D-5024 Puhlheim- Danzweiler (DE)**
Erfinder: **Harhausen, Ekkehard, Dr., Käulchensweg 32, D-5000 Köln 91 (DE)**

(74) Vertreter: **Sternagel, Hans- Günther, Dr., Patentanwälte Dr. Michael Hann Dr. H.- G. Sternagel Sander Aue 30, D-5060 Bergisch Gladbach 2 (DE)**

## Beschreibung

Gegenstand der Erfindung ist, ein Verfahren zur Herstellung eines neuen Lungensurfactants zu schaffen in Form eines Lyophilisats, einer Mischung von Dipalmitoylphosphatidylcholin, Dipalmitoylphosphatidylglycerol und einem Zucker. Das hergestellte Lungensurfactant kann nach Redispergieren in einer Pufferlösung zur Behandlung von Atemnotsyndromen verwendet werden.

An Atemnotsyndromen sterben mehr Neugeborene als an jeder anderen Krankheit. Die Ursache ist eine zu hohe Oberflächenspannung in den Alveolen, die die Lungen daran hindert, selbständig zu atmen. Sie beruht auf einem Mangel an Surfactant-Faktoren. Diese Substanz entsteht normalerweise kurz vor der Geburt. Die für die Atmung benötigten oberflächenaktiven Substanzen sind Phospholipide, die in den Alveoleren Zellen des Typs II im Rahmen des Phospholipid-Stoffwechsels gebildet werden. Sie sind in ungenügender Menge vorhanden, wenn das Kind zu früh geboren oder durch Kaiserschnitt entbunden wird, bevor die Wehen eingesetzt haben oder wenn die Mutter Diabetikerin ist.

In Mitteleuropa liegt die Rate des Atemnotsyndroms bei Frühgeborenen zwischen 15 und 20 %. Zur Prophylaxe und Therapie des Atemnotsyndroms hat man versucht, die Phospholipid-Synthese durch Glykocorticoide oder Bromhexinmetaboliten zu stimulieren. Die Therapie mit Glykocorticoiden ist mit einem hohen Risiko belastet und wird deshalb sehr zögernd angewendet. Die Therapie mit Bromhexinmetaboliten wurden aus verschiedenen Gründen wieder aufgegeben.

Es hat deshalb nicht an Versuchen gefehlt, ein möglichst natürliches Surfactantsystem zu finden, welches die notwendige Oberflächenspannung im Lungensystem wiederherstellt.

In US-A-3 594 476 ist beschrieben, Lungensurfactants in Aerosolform herzustellen, indem man Dipalmitoylphosphatidylcholin in einer wäßrigen Natriumchloridlösung dispergiert. Die erhaltenen Dispersionen sind jedoch nicht ausreichend stabil und haben eine ungenügende Oberflächenaktivität, so daß sie nicht zur therapeutischen Anwendung gelangten.

In GB-A-2 050 832 sind Lungensurfactantsysteme beschrieben, die aus Lungengeweben von Säugetieren gewonnen werden und die aus Phospholipiden, Cholesterin, Kohlenhydraten und Protein bestehen. Diese Präparate haben den Nachteil, daß sie nicht in gleichbleibender Zusammensetzung erhalten werden können und durch die Anwesenheit von Fremdeiweiß zur Bildung von Antikörpern gegen das Surfactant eiweiß führen können.

Als brauchbar haben sich Liposom-Suspensionen aus Dipalmitoylphosphatidylcholin und Dipalmitoylphosphatidylglycerol im Verhältnis 9 : 1 erwiesen (M. Obladen, und andere, Eur. J. Pediat. Vol. 131, Nr. 4, 219-228 (1979)). Diese Liposome wurden hergestellt durch Suspendieren bzw. Dispergieren von Gemischen aus Diplamitoylphosphatidylcholin und Dipalmitoylphosphatidylglycerol in wäßriger Lösung. Die so hergestellten Suspensionen zeigen jedoch keine ausreichende Stabilität und weisen eine ungleichmäßige Oberflächenaktivität auf.

Aufgabe der vorliegenden Erfindung ist es, ein Verfahren zur Herstellung eines Lungensurfactantsystems zu schaffen, das aus Dopalmitoylphosphatidylcholin und Dipalmitoylphosphatidylglycerol besteht und eine gleichbleibende Oberflächenaktivität besitzt sowie für längere Zeiträume lagerstabil ist.

Völlig überraschend wurde gefunden, daß man brauchbare stabile Lungensurfactantsysteme mit gleichbleibender Oberflächenaktivität herstellen kann, indem man Dipalmitoylphosphatidylcholin (DPPC) mit Dipalmithoylphosphatidylglycerol (DPPG) im Verhältnis 9 : 1 bis 8 : 2 in Eisessig löst, die erhaltene Lösung mit einem Zucker versetzt und anschließend gefriertrocknet.

Gegenstand der Erfindung ist daher ein Verfahren zum Herstellen eines redispergierbaren Lungensurfactants aus einer Mischung von Dipalmitoylphosphatidylcholin, Dipalmitoylphosphatidylglycerol, und üblichen Hilfsstoffen, dadurch gekennzeichnet, daß man 8 bis 9 Gewichsteile Dipalmitoylphosphatidylcholin und 1 bis 2 Gewichsteile Dipalmitoylphosphatidylglycerol jeweils in 10 bis 30 Gewichsteilen Eisessig auflöst, die Lösungen vereinigt und der vereinigten Lösung unter Rühren 9 bis 11 Gewichsteile Zucker hinzufügt und anschließend gefriertrocknet.

Man löst 8 - 9 Gewichsteile Dipalmitoylphosphatidylcholin und 2 - 1 Gewichsteile Dipalmitoylphosphatidylglycerol (DPPG) jeweils in 10 - 30 Gewichsteilen Eisessig bei 20 - 40° C, vereinigt die erhaltenen Einzellösungen unter Rühren und bringt dann den Zucker ein und zwar 1 Teil Zucker auf 1 Teil DPPC/DPPG-Gemisch.

Als geeignete Zucker können Glucose, Fructose oder Lactose zugegeben werden. Die erhaltene Lösung wird gegebenenfalls steril filtriert und anschließend nach üblichen Methoden gefriergetrocknet. Die erhaltenen Lyophilisate zeigen auch bei längerer Lagerung (1 Jahr) noch ausgezeichnete Stabilitäten. Das Lyophilisat wird vor Anwendung in einer Pufferlösung redispergiert. Die erhaltene homogene Dispersion kann intrapulmonal oder intratracheal verabreicht werden. Geeignete Pufferlösungen sind die dem Fachmann geläufigen Pufferlösungen, insbesondere Phosphatpuffer.

## Beispiel 1

45 mg Dipalmitoylphosphatidylcholin und 5 mg Dipalmitoylphosphatidylglycerol werden jeweils in 1 ml Eisessig (98 %) unter Erwärmen gelöst. Die klaren, warmen Einzellösungen werden unter Rühren zusammengegeben und 50 mg Glucose zugesetzt. Die Lösung wird in ein 10 ml Glasfläschchen eingetragen, lyophilisiert und die Glasflasche anschließend verschlossen.

Vor der Anwendung wird das Lyophilisat jeweils in 1,00 ml Trispuffer 0,35 % (Trispuffer 0,6 %, $H_2O$, Phosphatpuffer) redispergiert.

Messung der Oberflächenaktivität:

Die Messungen wurden mit einer modifizierten Wilhelmy-Waage durchgeführt.

Das aktive Material wurde als Dispersion auf die Oberfläche einer physiologischen Kochsalzlösung in einer Menge von 2,55 $\mu/cm^2$ Oberfläche bei einer Temperatur von 37° C gegeben.

Als Kriterium zur Beurteilung der Oberflächenaktivität wurde neben dem Stabilitätsindex (SI) auch die Hysteresiskurve miteinbezogen.

Es wurde ein Maximaloberflächenspanngsbereich von 61 x $10^{-5}$ - 68 · $10^{-5}$ Newton/cm (61 - 68 dyn/cm) und nach 50 - 75 % des komprimierbaren Oberflächenbereichs ein Mindestoberflächenspannungsbereich von 0 - $10^5$ Newton/cm (0 - 1 dyn/cm) erhalten. Der Stabilitätsindex betrug 1,95 - 2,00.

## Beispiel 2

Analog Beispiel 1 wurde eine Mischung aus 45 mg Dipalmitoylphosphatidylcholin und 10 mg Dipalmitoylphosphatidylglycerol eingesetzt.

## Beispiel 3

Analog Beispiel 1 wurde anstelle von Glucose 50 mg Lactose eingesetzt.

## Beispiel 4

Analog Beispiel 2 wurden anstelle von Glucose 50 mg Lactose eingesetzt.

## Patentansprüche

1. Verfahren zum Herstellen eines redispergierbaren Lungensurfactants aus einer Mischung von Dipalmitoylphosphatidylcholin, Dipalmitoylphosphatidylglycerol und üblichen Hilfsstoffen, dadurch gekennzeichnet, daß man 8 bis 9 Gewichtsteile Dipalmitoylphosphatidylcholin und 1 bis 2 Gewichtsteile Dipalmitoylphosphatidylglycerol jeweils in 10 bis 30 Gewichtsteilen Eisessig auflöst, die Lösungen vereinigt und der vereinigten Lösung unter Rühren 9 bis 11 Gewichtsteile Zucker hinzufügt und anschließend gefriertrocknet.

2. Verfahren nach Anspruch 1, dadurch gekennzeichnet, daß man als Zucker Glucose, Fructose und/oder Lactose verwendet.

## Claims

1. A method for producing a redispersible lung surfactant from a mixture of dipalmitoyl phosphatidyl choline, dipalmitoyl phosphatidyl glycerol and conventional adjuvants, characterized in that, 8 to 9 parts by weight of dipalmitoyl phosphatidyl choline and 1 to 2 parts by weight of dipalmitoyl phosphatidyl glycerol are individually dissolved in 10 to 30 parts by weight of glacial acetic acid, the solutions are combined and 9 - 10 parts by weight of sugar are added to the combined solution during stirring and subsequently freeze-dried.

2. Method according to claim 1, characterized in that, glucose, fructose and/or lactose are used as the sugar.

## Revendications

1. Procédé de préparation d'agents surfactifs pulmonaires pouvant être redispersés et consistant en un mélange de dipalmltylphosphatidylcholine, de dipalmitylphosphatidylglycérol et de substances auxiliaires usuelles, caractérisé en ce qu'on dissout 8 à 9 parties en poids de dipalmitylphosphatidylcholine et 1 à 2 parties en poids de dipalmitylphosphatiaylglycérol dans 10 à 30 parties en poids d'acide acétique glacial pour chacun, on combine les solutions et on ajoute à la solution combinée 9 à 11 parties en poids de sucre tout en agitant, puis finalement on lyophilise.

2. Procédé suivant la revendication 1, caractérisé en ce qu'on utilise comme sucre du glucose, du fructose et/ou du lactose.